# FASCICULE DE BREVET EUROPEEN

(11) **EP 0 785 919 B1**
(45) Date de publication et mention de la délivrance du brevet: **10.02.1999**
(21) Numéro de dépôt: 95933473.1
(22) Date de dépôt: 04.10.1995
(51) Int. Cl.: C07C 51/12, C07C 67/36

(54) **PREPARATION D'ACIDES CARBOXYLIQUES OU DES ESTERS CORRESPONDANTS PAR CARBONYLATION EN PRESENCE D'IRIDIUM**
HERSTELLUNG DER CARBONSÄUREN ODER IHRER ESTER DURCH CARBONYLIERUNG IN GEGENWART VON IRIDIUM
PREPARATION OF CARBOXYLIC ACIDS OR RELATED ESTERS BY CARBONYLATION IN THE PRESENCE OF IRIDIUM

(30) Priorité: 05.10.1994 FR 9412044
(43) Date de publication de la demande: 30.07.1997
(73) Titulaire: ACETEX CHIMIE, 92082 Paris La Défense 2 (FR)
(72) Inventeur: DENIS, Philippe, F-69150 Décines (FR); NOBEL, Dominique, F-30140 Salindres (FR); PERRON, Robert, F-69390 Charly (FR); SCHWARTZ, Joel, F-69300 Caluire (FR)
(74) Mandataire: Giraud, Françoise
(86) Numéro de dépôt international: FR9501283
(87) Numéro de publication internationale: WO9611179

(56) Documents cités:
- US-A- 3 772 380

## Description

La présente invention concerne un procédé de préparation d'acides carboxyliques comprenant (n+1) atomes de carbone, ou des esters correspondants, par carbonylation en phase liquide d'un alcool comprenant (n) atomes de carbone, en présence d'un catalyseur homogène comprenant de l'iridium.

Plus particulièrement le procédé selon l'invention permet d'obtenir une productivité élevée en acide carboxylique formé avec une sélectivité améliorée en ce produit.

Lors de la préparation d'acide carboxylique par réaction de monoxyde de carbone avec au moins un alcool, deux cycles catalytiques sont mis en oeuvre, le premier est constitué par la réaction de carbonylation proprement dite et le second par une réaction secondaire, appelée communément réaction de gaz à l'eau. Lors de cette réaction se forment des sous-produits gazeux comme notamment le dioxyde de carbone.

Ainsi, l'objectif à atteindre est bien évidemment de favoriser la première réaction par rapport à la seconde.

La carbonylation d'alcool, ou de tout autre composé carbonylable, en présence d'un système catalytique à base d'iridium, pour donner l'acide carboxylique ou l'ester correspondant est connue de l'homme de l'art. Cependant, de tels procédés sont très peu performants puisque les vitesses de carbonylation rapportées sont de l'ordre de 2 à 4 mol/h.l d'acide formé.

On a décrit dans la demande de brevet français no. 93 03734, un procédé de carbonylation du méthanol mettant en oeuvre un système catalytique comprenant de l'iridium. Ce procédé est caractérisé par le maintien d'une composition spécifique du mélange réactionnel. Dans le cas de l'obtention d'acide acétique à partir de méthanol, la composition comprend jusqu'à 10 % d'eau, d'alcool et d'iodure de méthyle ; jusqu'à 40% d'acétate de méthyle ; le reste étant constitué par l'acide acétique, employé comme solvant de la réaction. Les essais décrits ont été mis en oeuvre avec des pressions partielles en monoxyde de carbone relativement faibles, de l'ordre de 10 à 20 bar. Les performances de ce procédé sont nettement améliorées par rapport à celles des procédés antérieurs, et l'intérêt technique de ce dernier n'est pas remis en cause. Cependant, la sélectivité en sous-produits tels que le dioxyde de carbone reste élevée, puisqu'elle varie entre 1 et 2 %.

Il est par ailleurs décrit dans la demande de brevet français no. 94 05896, un procédé de carbonylation mettant en oeuvre un système catalytique comprenant de l'iridium et des iodures solubles dans le mélange réactionnel, dans les conditions de la réaction, choisis notamment parmi les iodures de métaux alcalins. Les performances, en terme de vitesse de carbonylation sont là encore améliorées par rapport aux procédés antérieurs, mais le problème de la sélectivité en dioxyde de carbone se pose toujours. En effet, les sélectivités en ce sous-produit varient entre 1 et 3 %.

De telles valeurs ne sont pas négligeables en ce sens qu'elles correspondent à des pertes relativement importantes de monoxyde de carbone ; pertes qui sont ressenties doublement. Tout d'abord, et d'une façon évidente, le monoxyde de carbone engagé est consommé pour autre chose que la formation de l'acide ou de l'ester. En outre, on constate une perte supplémentaire en monoxyde de carbone, due à la nécessité d'augmenter le nombre de purges et par conséquent d'augmenter les pertes en monoxyde de carbone entraîné lors de l'évacuation des gaz. En effet, l'apparition de sous-produits gazeux contribue à la diminution de la pression partielle en monoxyde de carbone dans le ciel gazeux du réacteur, avec pour résultat la nécessité d'augmenter le nombre de purges pour maintenir constante ladite pression partielle.

La présente demande a donc pour objet de pallier ce type d'inconvénients, au moins en conservant, voire en améliorant la vitesse de carbonylation de la réaction.

Il a été trouvé d'une façon totalement surprenante que l'on pouvait atteindre de tels objectifs en mettant en oeuvre la réaction de carbonylation en présence de teneurs élevées en ester et en iodure de méthyle, en combinaison avec une pression partielle importante en monoxyde de carbone.

Ainsi, le procédé selon l'invention consiste à faire réagir en phase liquide, le monoxyde de carbone avec au moins un alcool, en présence d'un système catalytique à base d'au moins un composé de l'iridium et d'au moins un promoteur halogéné ; procédé dans lequel on maintient dans le milieu, pendant la réaction, l'ester correspondant à l'acide carboxylique et à l'alcool précités, à une teneur variant entre 15 et 35 %, le promoteur halogéné à une teneur variant entre 10 % et 20 %, et une pression partielle en monoxyde de carbone comprise entre 40 et 200 bar.

Mais d'autres buts et avantages de la présente invention apparaîtront plus clairement à la lecture de la description qui va suivre.

Par la suite, et sauf indication contraire, les pourcentages indiqués sont exprimés en poids, rapportés au poids total du mélange réactionnel.

Ainsi qu'il a été dit précédemment, la réaction de carbonylation de l'invention est réalisée en présence d'un système catalytique à base d'au moins un composé de l'iridium et d'au moins un promoteur halogéné.

La réaction étant effectuée en phase liquide, le système catalytique se présente sous forme de composés solubles dans le mélange réactionnel.

Tous les composés de l'iridium, solubles ou pouvant être solubilisés dans le milieu réactionnel, dans les conditions de réalisation de l'invention, peuvent être utilisés. A titre d'exemple et sans intention de se limiter, sont susceptibles de convenir à la mise en oeuvre de l'invention, l'iridium à l'état métallique, les sels simples de ce métal, les oxydes ou encore les complexes de, coordination. Pour plus de détails, on pourra notamment se référer au brevet américain US 3 772 380 dans laquelle figure une liste indicative de tels composés.

Plus particulièrement, on utilise des sels simples d'iridium, comme les halogénures d'iridium, l'halogène étant choisi parmi le chlore, le brome ou de préférence l'iode.

Les oxydes d'iridium, de même que les complexes solubles de coordination de l'iridium, conviennent à la mise en oeuvre de l'invention. Dans cette dernière catégorie les composés les plus couramment employés sont ceux présentant des ligands choisis parmi le monoxyde de carbone ou une combinaison monoxyde de carbone/halogène, l'halogène étant choisi parmi le chlore, le brome ou plus particulièrement l'iode. Il n'est toutefois pas exclu d'utiliser des complexes solubles d'iridium dont les ligands sont choisis parmi des composés organo-phosphorés ou organo-azotés, par exemple.

Ces catalyseurs peuvent être obtenus par toute méthode connue de l'homme du métier.

Cependant, selon un mode particulièrement avantageux, on peut préparer une solution catalytique à partir d'un complexe carbonylé d'iridium, tel que Ir₄CO₁₂, par mise en contact dudit composé avec de l'acide iodhydrique et/ou un précurseur d'un tel acide, en présence d'un solvant.

Comme précurseur susceptible de libérer l'acide iodhydrique, on peut mentionner, à titre d'exemple, l'iode, les iodures d'alkyle en C₁-C₁₀, les iodures d'alcoyle en C₁-C₁₀, ou encore les iodures de métaux alcalins.

En ce qui concerne les solvants, tous les composés peuvent être utilisés dans la mesure où ils solubilisent l'acide iodhydrique ou son précurseur et le composé à base d'iridium obtenu. Plus particulièrement on met en oeuvre des solvants choisis parmi les acides carboxyliques ou les esters correspondants, obtenus par le procédé selon l'invention, ou l'eau ; ces composés étant utilisés seuls ou en mélange.

La mise en contact a lieu sous une pression totale comprise entre 1 et 10 bar, à une température au plus égale à la température d'ébullition du solvant précité, dans les conditions de la mise en contact.

L'opération peut être effectuée sous air, sous un gaz inerte ou encore sous monoxyde de carbone.

Un autre exemple de méthode avantageuse pour la préparation d'une solution catalytique convenable à la mise en oeuvre de l'invention, consiste à mettre en contact, en phase liquide, un ou plusieurs oxydes hydratés ou non d'iridium, avec de l'acide iodhydrique ou un composé susceptible de libérer de l'acide iodhydrique. L'acide iodhydrique peut être employé sous la forme d'un gaz, d'une solution, plus particulièrement aqueuse. Il peut encore être utilisé sous la forme d'un précurseur, tels que ceux mentionnés dans la précédente variante.

Plus particulièrement la quantité d'acide iodhydrique est telle que le rapport entre le nombre de moles d'acide iodhydrique au nombre de moles d'iridium, varie entre 1 et 100.

Cette variante de préparation d'une solution catalytique peut être mise en oeuvre sous air, sous un gaz inerte, sous monoxyde de carbone, ces gaz étant seuls ou en combinaison.

La concentration totale en iridium dans le mélange, engagé dans le procédé selon l'invention, est comprise entre 0,1 et 100 mmol/l.

Selon un mode de réalisation particulier, la concentration en iridium varie de 0,5 à 40 mmol/l et de préférence entre 1 et 25 mmol/l.

Le second constituant du système catalytique est un promoteur halogéné. Celui-ci peut se présenter sous la forme d'un halogène seul ou en combinaison avec d'autres éléments, comme par exemple l'hydrogène, un radical alkyle en C₁-C₁₀, un radical acyle en C₁-C₁₀, un radical aryle en C₆-C₁₀.

L'halogène est en général choisi parmi le chlore, le brome ou l'iode, ce dernier étant préféré.

Selon un mode de réalisation particulier de l'invention, le promoteur halogéné mis en oeuvre comprend, outre l'halogène, l'hydrogène ou un radical alkyle en C₁-C₁₀. Plus particulièrement, le promoteur utilisé dans l'invention comprend l'halogène et un radical alkyle en C₁-C₁₀.

De préférence, on effectue la réaction selon l'invention en présence d'un promoteur halogéné dont le radical correspond à celui de l'alcool utilisé comme réactif.

La teneur en promoteur halogéné dans le milieu est comprise entre 10 et 20%.

Selon un mode de réalisation particulier, la teneur en promoteur halogéné varie entre 10 exclu et 20 %.

Une variante de l'invention consiste à maintenir la teneur en promoteur halogéné dans le milieu réactionnel, entre 10 exclu et 15 %.

Le procédé selon l'invention est par ailleurs mis en oeuvre en présence de monoxyde de carbone. Celui-ci peut être utilisé sous forme pure ou diluée dans des gaz tels que l'hydrogène, le méthane, le dioxyde de carbone, ou tout autre type de gaz comme par exemple l'azote.

Selon un mode particulier de réalisation de l'invention, on utilise un monoxyde de carbone présentant une pureté d'au moins 99 %.

Le procédé selon l'invention est réalisé en maintenant pendant la réaction, une pression partielle en monoxyde de carbone comprise entre 40 et 200 bar.

Selon une première variante, la pression partielle est comprise entre 40 et 70 bar. Plus particulièrement, la pression partielle en monoxyde de carbone est comprise entre 50 et 70 bar.

Selon une seconde variante, la pression partielle en monoxyde de carbone est comprise entre 70 bar exclu et 200 bar. Sans pour autant se limiter à une telle altemative, l'emploi de telles pressions partielles rend possible la mise en oeuvre de la réaction à des températures plus basses que celles des procédés réalisés à des pressions plus faibles. Ceci représente notamment un avantage sur le plan de la corrosivité du milieu réactionnel.

Le procédé selon l'invention est réalisé en présence d'esters correspondant à la réaction de l'alcool mis en jeu dans la réaction, avec l'acide carboxylique présent dans le milieu réactionnel.

Selon une caractéristique du procédé de l'invention, la teneur en ester dans ledit milieu est comprise entre 15 et 35 %.

Une variante de réalisation particulière consiste à mettre en oeuvre la réaction en maintenant une quantité d'ester comprise entre 15 et 25 %.

Selon un mode de réalisation particulier, la teneur en ester est comprise entre 18 et 23 %. Il est à noter que cette variante convient particulièrement lorsque la réaction de carbonylation est effectuée dans le domaine des faibles pressions partielles en monoxyde de carbone, c'est-à-dire comprises entre 40 et 70 bar.

La réaction de carbonylation selon l'invention est effectuée en outre, en présence d'eau. La teneur en eau dans le milieu est comprise entre 0 exclu et 15 %.

Plus particulièrement, la teneur en eau dans le milieu est comprise entre 0 exclu et 10 %.

Ainsi que cela a été indiqué auparavant, on utilise en tant que réactif au moins un alcool comprenant un atome de carbone en moins par rapport à l'acide carboxylique fabriqué, ou à l'ester correspondant.

Parmi les réactifs convenables à la mise en oeuvre de la réaction, on peut citer les alcools saturés, présentant un à dix atomes de carbone. Ces alcools peuvent être mono- ou di- hydroxylés. A titre d'exemple de tels composés, on peut citer notamment le méthanol, l'éthanol, le propanol, le butanol, le 1,4-butanediol.

Selon un mode de réalisation préféré, les alcools utilisés sont choisis parmi les composés monohydroxylés.

Il est important de noter que l'alcool utilisé comme réactif peut être présent dans le milieu réactionnel en tant que tel ou sous une forme masquée. En effet, ledit alcool peut se trouver indifféremment sous la forme d'un dérivé halogéné et/ou d'un éther et/ou d'un ester.

Ainsi, la teneur en réactif dans le milieu réactionnel peut varier dans de larges limites, du fait des différentes espèces sous lesquelles le réactif peut se présenter.

Par conséquent, la teneur en alcool en tant que tel, dans le milieu réactionnel peut être comprise entre 0 et 10 %. De préférence, le milieu comprend une teneur en alcool comprise entre 0,1 et 5 %.

Selon une première mise en oeuvre de l'invention, on effectue la réaction en présence de composés ioniques solubles dans le milieu réactionnel et dont l'un des ions est l'iode (par la suite ces composés seront nommés iodures). Les iodures peuvent être introduits en tant que tels dans le milieu réactionnel mais aussi sous la forme de composés susceptibles de former des iodures solubles.

Ainsi, les iodures introduits dans ledit mélange, en tant que tels, sont choisis parmi les iodures minéraux ou organiques.

A titre d'iodures minéraux, on peut citer les iodures de métal alcalino-terreux ou alcalin, ces derniers étant préférés. Par exemple, l'iodure de potassium, l'iodure de lithium, l'iodure de sodium, conviennent à la réalisation de l'invention.

A titre d'iodures organiques, on peut citer les composés organiques, comprenant au moins un groupe organo-phosphoré et/ou au moins un groupe organo-azoté, réagissant avec des composés à base d'iode, pour donner des espèces ioniques renfermant cet halogène. A titre d'exemple, on peut mentionner l'iodure de triphényle phosphonium, l'iodure de N-méthyltriéthyle ammonium.

Parmi les composés susceptibles de former des iodures solubles dans le milieu réactionnel, conviennent notamment les carboxylates de métaux alcalins ou alcalino-terreux, tels que l'acétate de lithium notamment.

Plus particulièrement on effectue la réaction en présence d'une quantité d'iodures dépendant de la quantité d'iridium présent dans le milieu. Ainsi, cette quantité d'iodures introduite est telle que le rapport atomique iode (provenant des iodures) / iridium (exprimé en mole/mole), est maintenu à une valeur au plus égale à 10.

De préférence, le rapport atomique est au plus égal à 3.

Selon une seconde mise en oeuvre de l'invention, le procédé est réalisé en l'absence d'iodures solubles tels que définis ci-dessus.

Enfin, le procédé selon l'invention est effectué dans un solvant qui, de préférence, correspond à l'acide carboxylique formé par la réaction.

Ainsi que cela a été indiqué auparavant, la présente invention consiste à maintenir dans le milieu réactionnel, le promoteur halogéné, l'ester précité, une pression partielle de monoxyde de carbone, éventuellement les iodures, l'eau et l'acide carboxylique, dans les proportions venant d'être explicitées.

Par conséquent, la présente invention est plus particulièrement destinée à être mise en oeuvre en continu et les conditions stables de fonctionnement du procédé correspondent à la composition et aux proportions indiquées.

Lors du démarrage de la réaction, les divers composants sont introduits dans un réacteur approprié, muni de moyens d'agitation suffisants pour assurer le transfert gaz-liquide. Il est à noter que si le réacteur comprend de préférence des moyens d'agitation mécanique du mélange réactionnel, il n'est pas exclu d'opérer sans de tels moyens ; l'homogénéisation du mélange pouvant être réalisée par l'introduction du monoxyde de carbone dans le réacteur.

Les composants du milieu réactionnel sont introduits sans ordre préférentiel, sous leur forme propre et/ou sous la forme d'un ou plusieurs précurseurs.

Une première variante de l'invention consiste à introduire le promoteur halogéné décrit auparavant, tel quel, dans le mélange réactionnel.

Une seconde variante de mise en oeuvre, consiste à introduire ledit promoteur sous la forme d'au moins un précurseur.

Dans ce cas de figure, ce précurseur se présente généralement sous la forme d'un composé susceptible de libérer dans le milieu réactionnel le radical du promoteur halogéné précité, par réaction dudit précurseur avec un halogène, l'hydracide correspondant, ou un iodure, présent dans le milieu ou bien introduit à cet effet.

A titre d'exemple non limitatif de précurseurs convenables, on peut citer les composés choisis parmi les alcools de formule (1) ROH ; les éthers de formule (2) ROR' ou encore des esters de formules (3) R'COOR, utilisés seuls ou en mélange. Dans ces formules, les radicaux R et R', identiques ou différents, représentent chacun un radical alkyle en C₁-C₁₀, acyle en C₁-C₁₀, ou aryle en C₆-C₁₀ ; avec le radical R correspondant au radical du promoteur halogéné.

Ainsi, le méthanol, l'éthanol, le propanol, le butanol, le diméthyl éther, le diéthyl éther, l'oxyde d'éthylène, l'acétate de méthyle, sont notamment des précurseurs convenables du promoteur halogéné.

Habituellement la réaction de carbonylation est réalisée à une température comprise entre 150 et 250 °C. Selon une variante, la température de réaction varie entre 150 et 210°C.

La pression totale dans le réacteur est comprise entre 50 et 250 bar.

Il est à noter que le procédé selon l'invention peut être convenablement mis en oeuvre dans les installations exploitant les procédés classiques.

Ainsi, ces procédés sont habituellement constitués de trois zones. La première correspond à la zone de réaction, comprenant un réacteur sous pression. La seconde est celle de séparation de l'acide formé, ou de l'ester correspondant, par vaporisation partielle du mélange réactionnel. La partie vaporisée est ensuite envoyée dans la troisième zone, celle de purification de l'acide carboxylique ou de l'ester correspondant. La partie du mélange restée sous forme liquide dans la zone de vaporisation partielle, comprenant notamment le catalyseur, est recyclée au réacteur.

Selon un mode de réalisation particulier du procédé selon l'invention, le mélange réactionnel est purgé régulièrement des métaux de corrosion qu'il contient, dont notamment le fer, le molybdène, le chrome, le nickel. Cette opération est réalisée selon tout moyen connu de l'homme du métier, comme par exemple le traitement du mélange réactionnel par une résine échangeuse d'ions ou encore par précipitation du catalyseur et séparation de ce dernier, des métaux de corrosion, par filtration.

Le procédé selon l'invention convient à la fabrication de tout type d'acide carboxylique comprenant au minimum deux atomes de carbone, ou de l'ester correspondant. Ainsi, celui-ci peut être mis en oeuvre pour préparer l'acide propionique à partir de l'éthanol, l'acide succinique à partir de l'oxyde d'éthylène, l'acide adipique à partir du 1,4-butanediol, ou les esters correspondants à ces acides.

Cependant, ce procédé convient tout particulièrement à l'obtention d'acide acétique et/ou d'acétate de méthyle à partir de méthanol.

Selon un mode de réalisation préféré de l'invention, le procédé est mis en oeuvre avec, de l'iodure de méthyle, de l'acétate de méthyle, éventuellement des iodures précités, de l'eau et de l'acide acétique en tant que solvant, outre le méthanol et le monoxyde de carbone.

Des exemples concrets mais non limitatifs de l'invention vont maintenant être présentés.

### EXEMPLES

Les essais suivants ont été réalisés en continu dans un autoclave de 300 cm³ muni d'un moyen d'agitation mécanique et de moyens d'introduction des réactifs.

La solution catalytique a été obtenue de la façon suivante :
On introduit dans un ballon en verre 10 g de Ir₄(CO)₁₂, 50 g d'acide iodhydrique en solution à 57 % dans l'eau et 290 g d'acide acétique.

Le mélange est ensuite chauffé à reflux sous agitation et sous air, pendant 4 heures.

Les introductions de méthanol, d'iodure de méthyle et d'eau sont réglées de telle sorte que les teneurs des différents composants, de même que la pression partielle en monoxyde de carbone et la pression totale, soient maintenues comme indiquées dans le tableau suivant.

A noter que la teneur en eau reste comprise entre 6 et 8 % et que la teneur en méthanol est comprise entre 0,1 et 2 %. Par ailleurs, le complément à 100 % est constitué par l'acide acétique employé comme solvant de la réaction.

Le temps de séjour dans le réacteur est d'environ 10 minutes.

La température est maintenue à 190°C.

A la sortie de l'autoclave, le mélange réactionnel est dégazé et refroidi.

Le mélange et les gaz sont analysés par chromatographie en phase gazeuse.

| **ESSAIS** | **CH**_{**3**}**CO**_{**2**}**CH**_{**3**} **(% poids)** | **CH**_{**3**}**I (% poids)** | **P**_{**CO**}**/P**_{**tot**} **(bar)** | **Ir (ppm)** | **V**_{**carb**} **(mol/h.l)** | **sélectivité CO**_{**2**}**(%)** |
|---|---|---|---|---|---|---|
| **comparatif A** | 10 | 4 | 18/30 | 680 | 6,2 | 2,2 |
| **comparatif B** | 11 | 11 | 40/52 | 800 | 8,4 | 1,4 |
| **comparatif C** | 18 | 3,6 | 18/30 | 640 | 8,6 | 1,4 |
| **D** | 23 | 11 | 40 / 52 | 700 | 16 | 0,6 |

V_{carb} représente la vitesse de carbonylation. Elle est obtenue par mesure du débit de consommation du CO en tenant compte, en outre, de la quantité de ce gaz engagée dans la formation de CO₂.

La vitesse de formation de CO₂ est contrôlée par mesure du débit de formation du CO₂. Cette valeur permet de calculer la sélectivité en CO₂ qui correspond au rapport de la vitesse de formation du CO₂ et de la vitesse de carbonylation.

Ce tableau indique que l'on n'obtient pas de performances améliorées en terme de vitesse de carbonylation et de sélectivité lorsque l'un ou plusieurs des paramètres de la réaction se trouvent en dehors des caractéristiques de l'invention.

## Revendications

1. Procédé de préparation d'acides carboxyliques présentant (n+1) atomes de carbone, ou des esters correspondants, par réaction en phase liquide, de monoxyde de carbone avec au moins un alcool présentant (n) atomes de carbone, en présence d'un système catalytique à base d'au moins un composé de l'iridium et d'au moins un promoteur halogéné, caractérisé en ce que l'on maintient dans le milieu, pendant la réaction, l'ester correspondant à l'acide carboxylique et à l'alcool précités, à une teneur variant entre 15 et 35 %, le promoteur halogéné à une teneur variant entre 10 % et 20 %, et une pression partielle en monoxyde de carbone comprise entre 40 et 200 bar.

2. Procédé selon la revendication précédente, caractérisé en ce que l'on maintient une pression partielle en monoxyde de carbone comprise entre 40 et 70 bar, et de préférence entre 50 et 70 bar.

3. Procédé selon la revendication 1, caractérisé en ce que l'on maintient une pression partielle en monoxyde de carbone comprise entre 70 bar exclu et 200 bar.

4. Procédé selon l'une quelconque des revendications précédentes, caractérisé en ce que l'on maintient une teneur en promoteur halogéné comprise entre 10 exclu et 20 %, et de préférence comprise entre 10 exclu et 15 %.

5. Procédé selon l'une quelconque des revendications précédentes, caractérisé en ce que l'on maintient une teneur en ester comprise entre 15 et 25 %.

6. Procédé selon l'une quelconque des revendications précédentes, caractérisé en ce que l'on maintient une teneur en ester comprise entre 18 et 23 %.

7. Procédé selon l'une quelconque des revendications précédentes, caractérisé en ce que l'on maintient une teneur en eau comprise entre 0 exclu et 15 %, de préférence comprise entre 0 exclu et 10 %.

8. Procédé selon l'une quelconque des revendications précédentes, caractérisé en ce que l'on effectue la réaction en présence d'au moins un composé ionique soluble dans le mélange réactionnel et dont l'un des ions est l'iode.

9. Procédé selon la revendication précédente, caractérisé en ce que l'on maintient une teneur en iode provenant des composés ioniques solubles telle que le rapport iode / iridium est au plus égal à 10.

10. Procédé selon l'une des revendications précédentes, caractérisé en ce que l'on prépare l'acide acétique par réaction du méthanol avec du monoxyde de carbone, en présence d'iodure de méthyle, d'acétate de méthyle, éventuellement d'au moins un composé ionique soluble, d'eau, l'acide acétique constituant le solvant de la réaction.

## Patentansprüche

1. Verfahren zur Herstellung von Carbonsäuren, die (n+1) Kohlenstoffatome aufweisen, oder entsprechender Ester durch Umsetzung von Kohlenmonoxid mit mindestens einem Alkohol, der (n) Kohlenstoffatome aufweist, in flüssiger Phase in Gegenwart eines katalytischen Systems auf Basis mindestens einer lridiumverbindung und mindestens eines halogenierten Promotors, dadurch gekennzeichnet, daß man in dem Medium während der Umsetzung den Ester, welcher der obengenannten Carbonsäure und dem obengenannten Alkohol entspricht, bei einem Gehalt, der zwischen 15 und 35 % variiert, den halogenierten Promotor bei einem Gehalt, der zwischen 10 und 20 % variiert, hält und einen Partialdruck an Kohlenmonoxid zwischen 40 und 200 bar aufrechterhält.

2. Verfahren nach dem vorhergehenden Anspruch, dadurch gekennzeichnet, daß man einen Partialdruck an Kohlenmonoxid zwischen 40 und 70 bar, vorzugsweise zwischen 50 und 70 bar, aufrechterhält.

3. Verfahren nach Anspruch 1, dadurch gekennzeichnet, daß man einen Partialdruck an Kohlenmonoxid zwischen 70 bar (wobei dieser Wert ausgeschlossen ist) und 200 bar aufrechterhält.

4. Verfahren nach einem der vorhergehenden Ansprüche, dadurch gekennzeichnet, daß man einen Gehalt an halogeniertem Promotor zwischen 10 % (wobei dieser Wert ausgeschlossen ist) und 20 %, vorzugsweise zwischen 10 % (wobei dieser Wert ausgeschlossen ist) und 15 %, aufrechterhält.

5. Verfahren nach einem der vorhergehenden Ansprüche, dadurch gekennzeichnet, daß man einen Estergehalt zwischen 15 und 25 % aufrechterhält.

6. Verfahren nach einem der vorhergehenden Ansprüche, dadurch gekennzeichnet, daß man einen Estergehalt zwischen 18 und 23 % aufrechterhält.

7. Verfahren nach einem der vorhergehenden Ansprüche, dadurch gekennzeichnet, daß man einen Wassergehalt zwischen 0 % (wobei dieser Wert ausgeschlossen ist) und 15 %, vorzugsweise zwischen 0 % (wobei dieser Wert ausgeschlossen ist) und 10 %, aufrechterhält.

8. Verfahren nach einem der vorhergehenden Ansprüche, dadurch gekennzeichnet, daß man die Umsetzung in Gegenwart mindestens einer ionischen Verbindung durchführt, die in der Reaktionsmischung löslich ist und bei der eines der Ionen lod ist.

9. Verfahren nach dem vorhergehenden Anspruch, dadurch gekennzeichnet, daß man einen solchen Gehalt an lod, das aus löslichen ionischen Verbindungen stammt, aufrechterhält, daß das Verhältnis Iod/Iridium ≤ 10 ist.

10. Verfahren nach einem der vorhergehenden Ansprüche, dadurch gekennzeichnet, daß man Essigsäure herstellt durch Umsetzung von Methanol mit Kohlenmonoxid in Gegenwart von Methyliodid, Methylacetat, gegebenenfalls mindestens einer löslichen ionischen Verbindung und von Wasser, wobei die Essigsäure das Reaktions-Lösungsmittel darstellt.

## Claims

1. Method for preparing carboxylic acids having (n+1) carbon atoms, or corresponding esters, by reaction of carbon monoxide with at least one alcohol having (n) carbon atoms, in the liquid phase and in the presence of a catalytic system based on at least one iridium compound and at least one halogenated promoter, characterised in that the ester corresponding to the above-mentioned carboxylic acid and alcohol is maintained in the medium during the reaction at a content between 15 and 35%, the halogenated promoter at a content between 10% and 20%, and a partial carbon monoxide pressure is maintained between 40 and 200 bar.

2. Method according the preceding claim, characterised in that a partial carbon monoxide pressure is maintained between 40 and 70 bar, and preferably between 50 and 70 bar.

3. Method according to claim 1, characterised in that a partial carbon monoxide pressure is maintained between 70 bar (excluded) and 200 bar.

4. Method according to any one of the preceding claims, characterised in that a halogenated promoter content is maintained between 10 (excluded) and 20%, and preferably between 10 (excluded) and 15%.

5. Method according to any one of the preceding claims, characterised in that an ester content is maintained between 15 and 25%.

6. Method according to any one of the preceding claims, characterised in that an ester content is maintained between 18 and 23%.

7. Method according to any one of the preceding claims, characterised in that a water content is maintained between 0 (excluded) and 15%, preferably between 0 (excluded) and 10%.

8. Method according to any one of the preceding claims, characterised in that the reaction is carried out in the presence of at least one ionic compound which is soluble in the reaction mixture and of which one of the ions is iodine.

9. Method according to the preceding claim, characterised in that a content of iodine originating from the soluble ionic compounds is maintained such that the iodine/iridium ratio is at the most equal to 10.

10. Method according to one of the preceding claims, characterised in that acetic acid is prepared by reaction of methanol with carbon monoxide in the presence of methyl iodide, methyl acetate, optionally at least one soluble ionic compound, water, and acetic acid which constitutes the solvent of the reaction.
